# EUROPÄISCHE PATENTSCHRIFT

(11) **EP 4 324 494 B1**
(45) Veröffentlichungstag und Bekanntmachung des Hinweises auf die Patenterteilung: **21.05.2025**
(21) Anmeldenummer: 23184210.5
(22) Anmeldetag: 07.07.2023
(51) Int. Cl.: A61M 1/00, A61B 17/32, A61B 17/16, A61B 10/02

(54) **SAMMELVORRICHTUNG**
COLLECTING DEVICE
DISPOSITIF COLLECTEUR

(30) Priorität: 19.08.2022 DE 102022121041
(43) Veröffentlichungstag der Anmeldung: 21.02.2024
(73) Patentinhaber: H & B Electronic GmbH & Co. KG, 75392 Deckenpfronn (DE)
(72) Erfinder: MORLOK, Tobias, 71159 Mötzingen (DE); HEIMSCH, Jochen, 70734 Fellbach (DE); REMY, Nico, 72070 Tübingen (DE); BAYERBACH, Jan, 75365 Calw (DE)
(74) Vertreter: Wacker, Jost Oliver

(56) Entgegenhaltungen:
- WO-A1-2014/143456
- CN-A- 107 744 404
- CN-B- 104 161 562
- US-A1- 2003 130 594
- US-A1- 2005 288 605
- US-A1- 2016 278 790
- US-A1- 2021 196 865
- US-A1- 2022 047 283

## Beschreibung

Die Erfindung betrifft eine Sammelvorrichtung für eine Gewebeentnahmevorrichtung, wie insbesondere eines Shavers, nach dem Oberbegriff des Anspruchs 1. Dabei dient die Sammelvorrichtung zum Zurückhalten und Sammeln eines mittels des Shavers von einem Patienten entnommenen Gewebematerials, wie insbesondere eines autologen Knorpelgewebes eines Gelenks oder Knochenmaterials. Das gesammelte Gewebematerial soll hierbei unter Vermischung mit einem Medium, wie insbesondere einem Hydrogel oder Blutserum, zur Aufbereitung und Herstellung einer insbesondere autologen Applikatormasse dienen. Die Sammelvorrichtung weist hierzu einen shaverseitigen Strömungsanschluss, der mit einer Gewebeabführung der Gewebeentnahmevorrichtung strömungsmäßig verbindbar ist, einen unterdruckseitigen Strömungsanschluss, der mit einer Unterdruckvorrichtung strömungsmäßig verbindbar ist, und einen Verbindungspfad auf, der zwischen dem shaverseitigen Strömungsanschluss und dem unterdruckseitigen Strömungsanschluss ausbildbar ist. Über diesen Verbindungspfad kann dabei ein Fluidstrom vom shaverseitigen Strömungsanschluss zum unterdruckseitigen Strömungsanschluss ausgebildet werden, in dem das abgetragene Gewebematerial von der Gewebeentnahmevorrichtung weg transportiert wird. Zudem ist ein Sammelbehälter vorgesehen, der im Verbindungspfad angeordnet werden kann und der zur Aufnahme von aus dem Fluidstrom abgetrenntem Gewebematerial dient. Dabei kann zwischen dem shaverseitigen Strömungsanschluss und dem unterdruckseitigen Strömungsanschluss ein vom Sammelbehälter getrennter Bypasspfad ausgebildet werden. Hierdurch kann der Fluidstrom zwischen den beiden Strömungsanschlüssen auch unabhängig vom Sammelbehälter ausgebildet werden.

Aus US 2021/0251647 A1 ist eine Vorrichtung zum Sammeln von Gewebe bekannt, die einen shaverseitigen Strömungsanschluss und einen unterdruckseitigen Strömungsanschluss aufweist. Dazwischen bildet die Sammelvorrichtung einen Verbindungspfad in Form eines mehrteiligen, hülsenförmigen Sammelbehälters, in dem ein Filterteil mit mehreren Filterelementen mit unterschiedlicher Durchlässigkeit aufgenommen ist. Zwei Gehäuseteile des Sammelbehälters können dabei voneinander getrennt werden, um auf das vom Filterteil zurückgehaltene Gewebematerial zugreifen zu können.

Nachteilig an der bekannten Sammelvorrichtung ist, dass diese zur Entnahme des Gewebematerials relativ umständlich zerlegt werden muss und der betreffende Shaver während einer solchen Gewebeentnahme auch nicht betrieben werden kann.

CN107744404A beschreibt eine Sammelvorrichtung für Patellagewebe, das einen Sammelbehälter zwischen einem Shaver und einem Unterdruckgerät aufweist. Um den Behälter während einer Anwendung leeren beziehungsweise tauschen zu können, ist zudem eine Bypassleitung vorgesehen. Zur Aktivierung des Bypasses ist am Einlass und am Auslass des Sammelbehälters sowie in der Bypassleitung jeweils ein Schließventil vorgesehen. Die Steuerung der Ventile kann dabei in Abhängigkeit eines Messsensors erfolgen, der in einer weiteren parallelen Messleitung vorgesehen ist.

US 2022/047283 A1 offenbart eine Lithotripsie-Vorrichtung zur Zertrümmerung von Steinen mit einem Handstück, an dem eine mit einer Stoßenergie versorgbare Sonde gelagert ist und durch den hindurch ein Saugkanal zu einem Gehäuse mit einer Fangvorrichtung geführt ist. Die Fangvorrichtung weist dabei in einer Ausführungsform eine Aufnahme für einen Auffangbehälter und eine um die Aufnahme herum geführte Bypass-Leitung mit zwei Zweiwegeventilen auf. Bei eingesetztem Auffangbehälter befinden sich die Zweiwegeventile in einer ersten Stellung, in der sie einen Strömungspfad durch den Auffangbehälter freigeben. Bei abgenommenem Behälter befinden sich die Zweiwegeventile dagegen in einer zweiten Stellung, in der sie einen Strömungspfad durch die Bypass-Leitung freigeben. Die Zweiwegeventile sind dabei in die zweite Stellung vorgespannt und werden beim Einsetzen des Auffangbehälters, über einen an diesem vorgesehenen Aktuator in die erste Stellung umgeschaltet.

Die Aufgabe der Erfindung ist es, bei einer gattungsgemäßen Sammelvorrichtung die genannten Nachteile zu vermeiden und eine leichtere und schnellere Entnahme des zurückgehaltenen Gewebes sowie einen komfortableren Betrieb der zugehörigen Gewebeentnahmevorrichtung zu ermöglichen.

Diese Aufgabe wird durch eine Sammelvorrichtung mit den Merkmalen des Anspruchs 1 gelöst. Dabei sind der shaverseitige Strömungsanschluss und der unterdruckseitige Strömungsanschluss an einem Anschlussgehäuse angeordnet, an dem der Sammelbehälter abnehmbar gehalten ist. Hierdurch ist es möglich, den Sammelbehälter zu Entnahme des zurückgehaltenen Gewebematerials vom Anschlussgehäuse und damit von der übrigen Gewebesammelanordnung zu trennen, die aus der Gewebeentnahmevorrichtung, dem Anschlussgehäuse, der Unterdruckvorrichtung und den strömungsmäßigen Verbindungen zwischen diesen besteht. Hierdurch werden eine besonders einfache und komfortable Entnahme und Aufbereitung des zurückgehaltenen Gewebematerials ermöglicht. Hierbei ist es möglich, die Gewebeentnahmevorrichtung auch während einer Entnahme von zurückgehaltenem Gewebematerial aus dem Sammelbehälter zu betreiben, da das vom Shaver abgetragene Gewebematerial auch weiterhin über einen Fluidstrom abtransportiert werden kann. Dabei ist eine Umschalteinrichtung vorgesehen, über die der shaverseitige Strömungsanschluss wahlweise über den Verbindungspfad oder den Bypasspfad mit dem unterdruckseitigen Strömungsanschluss verbunden werden kann. Auf diese Weise ist die Sammelvorrichtung zwischen einem Sammelbetrieb und einem reinen Absaugbetrieb umschaltbar, in dem das vom Shaver abgetragene Gewebe abtransportiert werden kann, ohne gesammelt zu werden. Hierdurch ist es wiederum möglich, den Sammelbehälter zu entleeren, während der Shaver parallel dazu im reinen Absaugbetrieb weiter betrieben wird.

Dabei ist die Umschalteinrichtung vorzugsweise durch ein Zweiwegeventil gebildet, um ohne wesentliche Unterbrechung ein schnelles Umschalten zwischen Sammelbetrieb und Absaugbetrieb zu ermöglichen.

Zudem ist es günstig, wenn die Umschalteinrichtung über ein Drehglied umgeschaltet werden kann, um eine besonders komfortable Bedienung, insbesondere mit einer Hand zu ermöglichen. Alternativ zum Drehglied kann die Umschalteinrichtung dabei auch ein Schiebeglied oder jede andere bekannte und zum Umschalten eines Zweiwegeventils geeignete Handhabe aufweisen.

Dabei ist es besonders günstig, wenn der Bypasspfad innerhalb des Anschlussgehäuses ausgebildet ist, so dass die übrige Gewebesammelanordnung auch ohne den Sammelbehälter im reinen Absaugbetrieb betrieben werden kann.

Bevorzugterweise bildet der Sammelbehälter zwischen einer Zutrittsöffnung und einer Austrittsöffnung einen Strömungsabschnitt des Verbindungspfades, in dem eine durch eine Siebeinrichtung begrenzte Sammelaufnahme vorgesehen ist. Hierdurch kann im Sammelbetrieb der Verbindungspfad über den Sammelbehälter aufgebaut und das Gewebe dabei in einfacher Weise und über eine relativ große Filterfläche aus dem Verbindungspfad abgetrennt werden.

Dabei ist die Siebeinrichtung vorzugsweise durch eine in Strömungsrichtung vor der Austrittsöffnung angebrachte ebene Siebfläche gebildet. Durch die ebene Ausbildung der Siebfläche wird dabei eine einfachere Entfernung von während des Sammelbetriebes anhaftenden Gewebepartikeln beziehungsweise eines anhaftenden Filterkuchens ermöglicht.

Ferner ist die Sammelaufnahme vorteilhafterweise über einen transparenten Bereich des Sammelbehälters einsehbar, wie beispielsweise durch ein Fenster oder eine transparente Wand des Sammelbehälters. Auf diese Weise kann im Verlauf des Sammelbetriebes in einfacher Weise festgestellt werden, ob eine ausreichende Menge des jeweiligen Gewebematerials gesammelt wurde. Zusätzlich kann hierzu an dem transparenten Bereich eine Füllstandskala vorgesehen sein, die eine Abschätzung der Menge des gesammelten Materials erleichtert.

Ferner ist es von Vorteil, wenn an dem abnehmbaren Sammelbehälter ein Schließelement gehalten ist, das zwischen einer ersten Offenstellung, in der die Zutrittsöffnung der Sammelaufnahme freigegeben ist, und einer Schließstellung verstellbar ist, in der die Zutrittsöffnung verschlossen ist. Hierdurch kann der Sammelbehälter über das Schließelement einerseits geöffnet werden, um in der am Anschlussgehäuse angebrachten Stellung den Verbindungspfad für den Sammelbetrieb freizugeben. Andererseits kann der Sammelbehälter über das Schließelement geschlossen werden, um im getrennten Zustand des Sammelbehälters vom Anschlussgehäuse einen ungewollten Austritt von zurückgehaltenem Gewebematerial beziehungsweise dessen Kontamination durch eintretende Verunreinigungen zu vermeiden.

Dabei ist es günstig, wenn das Schließelement beim Anbringen des Sammelbehälters am Anschlussgehäuse selbsttätig in die erste Offenstellung verbringbar ist, um die Freigabe des Verbindungspfades bei angebrachtem Sammelbehälter beziehungsweise im Sammelbetrieb zu gewährleisten.

Zudem ist es günstig, wenn das Schließelement beim Abnehmen vom Sammelbehälter selbsttätig in die Schließstellung verbringbar ist, um während des Trennens und im getrennten Zustand des Sammelbehälters vom Anschlussgehäuse einen ungewollten Austritt des enthaltenen Gewebematerials zu verhindern.

Hierbei weist das Schließelement vorteilhafterweise ein Stellelement auf, über das das Schließelement beim Anbringen des Sammelbehälters durch Zusammenwirken mit dem Anschlussgehäuse in die erste Offenstellung verstellbar ist. Beispielsweise ist das Stellelement durch einen an das Anschlussgehäuse anlegbaren und dadurch gegenüber dem Sammelgehäuse verlagerbaren Kontaktnocken gebildet. Hierdurch kann die selbsttätige Verlagerung des Schließelementes in die erste Offenstellung mit besonders einfachen Mitteln und geringen Herstellungskosten umgesetzt werden.

Zudem ist es von Vorteil, wenn das Schließelement in die Schließstellung vorgespannt ist. Hierdurch kann der Sammelbehälter sowohl während des Abnehmens vom Anschlussgehäuse als auch im vollständig getrennten Zustand von diesem sicher verschlossen werden, um einen versehentlichen Austritt und/oder eine Verunreinigung des gesammelten Gewebematerials zu vermeiden.

Vorteilhafterweise kann die Sammelaufnahme zudem in einer zweiten Offenstellung des Schließelementes mittels eines Kolbens entleert werden. Hierdurch wird eine besonders komfortable und vollständige Entnahme des gesammelten Gewebematerials aus dem Sammelbehälter heraus ermöglicht.

Hierbei ist es günstig, wenn der Sammelbehälter eine vom Schließelement verschließbare Behälteröffnung aufweist und der Kolben an einer vom Schließelement abgewandten Seite der Sammelaufnahme gehalten und entlang der Sammelaufnahme in Richtung der Behälteröffnung verlagerbar ist. Auf diese Weise kann die Sammelaufnahme in der zweiten Offenstellung des Schließelementes mittels des Kolbens in komfortabler Weise und vollständig entleert werden.

Ferner ist es von Vorteil, wenn der Kolben dabei entlang einer Innenoberfläche der Siebeinrichtung verlagerbar ist, wodurch auch zurückgehaltene Gewebepartikel, die an der Siebeinrichtung anhaften, in komfortabler Weise entfernt werden können, um das gesammelte Gewebematerial möglichst vollständig für eine Aufbereitung beziehungsweise eine spätere Verwendung zur Verfügung stellen zu können.

Es wird darauf hingewiesen, dass alle oben beschriebenen Merkmale des erfindungsgemäßen Gegenstandes untereinander austauschbar beziehungsweise kombinierbar sind, sofern ein Austausch oder eine Kombination derselben aus technischen Gründen nicht ausgeschlossen ist.

In den Figuren ist eine beispielhafte Ausführungsform der Erfindung dargestellt. Es zeigen:
- Figur 1: eine Gewebesammelanordnung mit einer erfindungsgemäßen Sammelvorrichtung,
- Figur 2: einen Längsschnitt durch die Sammelvorrichtung nach Figur 1 mit einem hergestellten Verbindungspfad,
- Figur 3: einen Längsschnitt durch die Sammelvorrichtung nach Figur 1 mit einem hergestellten Bypasspfad und
- Figur 4: eine explodierte Ansicht eines Sammelbehälters der Sammelvorrichtung nach Figur 1.

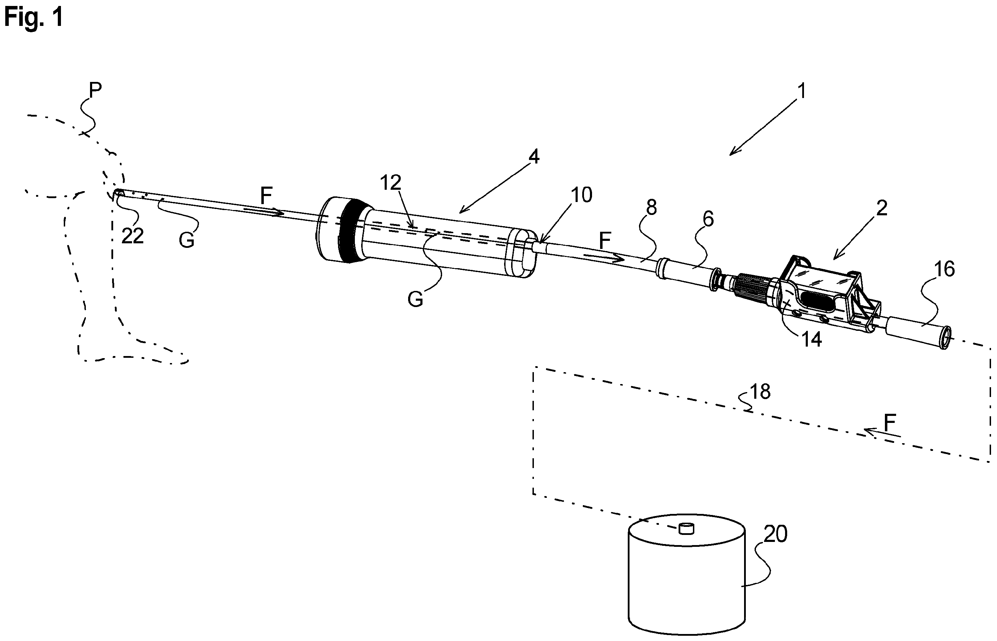

Fig. 1 zeigt eine Gewebesammelanordnung 1 mit einer Sammelvorrichtung 2, einer Gewebeentnahmevorrichtung 4 und einer Unterdruckvorrichtung 20. Die Gewebeentnahmevorrichtung 4 ist dabei in Form eines Shavers ausgebildet, mittels dem Gewebematerial G, wie insbesondere Knorpelgewebe oder Knochenmaterial, von einem Patienten P entnommen werden kann. Die Entnahme erfolgt dabei beispielsweise an einem Knie, einem Schultergelenk oder an einem sonstigen Knochen. Das hierbei gewonnene Gewebematerial G dient zur späteren Herstellung einer insbesondere autologen Applikatormasse, die bei der Behandlung eine Läsion beziehungsweise beim Aufbau von Gewebe beziehungsweise Knochen an einem Defekt des Patienten P verwendet werden kann.

Zum Sammeln des Gewebematerials G weist die Sammelvorrichtung 2 einen shaverseitigen Strömungsanschluss 6 auf, der über eine Verbindungsleitung 8 strömungsmäßig mit einem Abgabeanschluss 10 einer Gewebeabführung 12 der Gewebeentnahmevorrichtung 4 verbunden ist. Zudem weist die Sammelvorrichtung 2 einen mit dem shaverseitigen Strömungsanschluss 6 über einen Verbindungspfad 14 verbindbaren unterdruckseitigen Strömungsanschluss 16 auf, der über eine Anschlussleitung 18 mit einer Unterdruckvorrichtung 20 strömungsmäßig verbunden ist.

Sobald mittels der Unterdruckvorrichtung 20 ein Unterdruck an der Anschlussleitung 18 angelegt wird, wird dadurch ein Fluidstrom F von der Gewebeentnahmevorrichtung 4 über die Sammelvorrichtung 2 zur Unterdruckvorrichtung 20 erzeugt. Über die Gewebeabführung 12 kann dabei das von einem Werkzeug 22 abgetragene Gewebematerial G im Fluidstrom F zum Abgabeanschluss 10 transportiert werden, über den es weiter zur Sammelvorrichtung 2 geleitet wird.

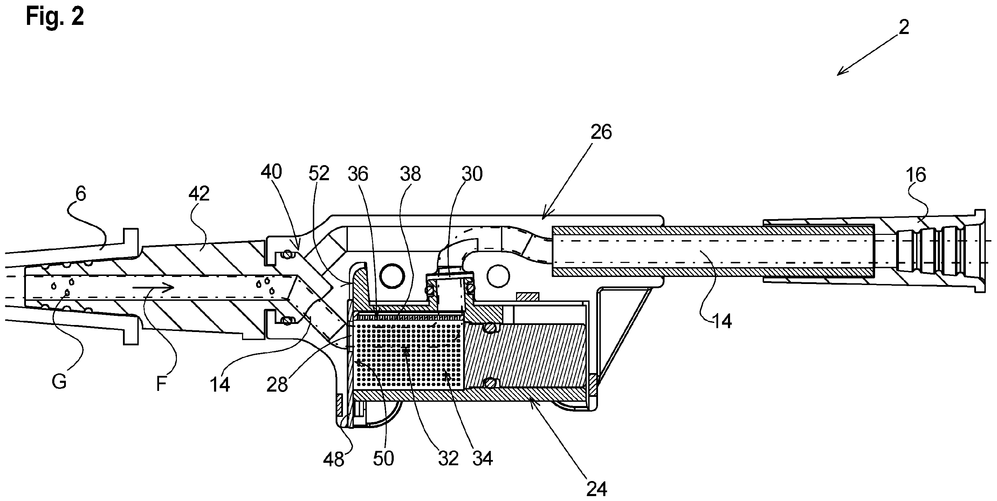

Wie insbesondere aus Figur 2 zu entnehmen ist, weist die Sammelvorrichtung 2 einen Sammelbehälter 24 auf, der an einem Anschlussgehäuse 26 gehalten ist. Der Sammelbehälter 24 bildet dabei zwischen einer Zutrittsöffnung 28 und einer Austrittsöffnung 30 einen Strömungsabschnitt 32, der durch eine Sammelaufnahme 34 verläuft. Die Sammelaufnahme 34 ist hierbei teilweise durch eine Siebeinrichtung 36 begrenzt, die in Strömungsrichtung des Fluidstroms F wenigstens eine ebene Siebfläche 38 vor der Austrittsöffnung 30 aufweist.

Wie aus Figur 2 ferner zu entnehmen ist, verläuft der Verbindungspfad 14 vom shaverseitigen Strömungsanschluss 6 über den Strömungsabschnitt 32 zum unterdruckseitigen Strömungsanschluss 16. Auf diese Weise kann das im Fluidstrom mitgeführte Gewebematerial G mittels der Siebeinrichtung 36 abgetrennt und in der Sammelaufnahme 34 gesammelt werden.

Zwischen dem shaverseitigen Strömungsanschluss 6 und dem Sammelbehälter 24 ist zudem ein als Umschalteinrichtung 40 fungierendes Zweiwegeventil vorgesehen, das zwischen einer Verbindungsstellung und einer Bypassstellung umschaltbar ist. Die Umschalteinrichtung 40 weist hierzu ein Stellglied 42 auf, das wie dargestellt, als Drehglied ausgebildet sein kann. Alternativ hierzu kann das Stellglied 42 auch als Schiebeglied oder in jeder anderen bekannten und zur Umschaltung eines Zweiwegeventils geeigneten Form ausgebildet sein (nicht dargestellt).

In jedem Fall ist in der in Figur 2 dargestellten Verbindungsstellung des Zweiwegeventils 40 der shaverseitige Strömungsanschluss 6 über den Verbindungspfad 14 mit dem unterdruckseitigen Strömungsanschluss 16 verbunden, so dass die Gewebesammelanordnung 1 mit der Sammelvorrichtung 2, der Gewebeentnahmevorrichtung 4 und der Unterdruckvorrichtung 20 in einem Sammelbetrieb verwendet werden kann.

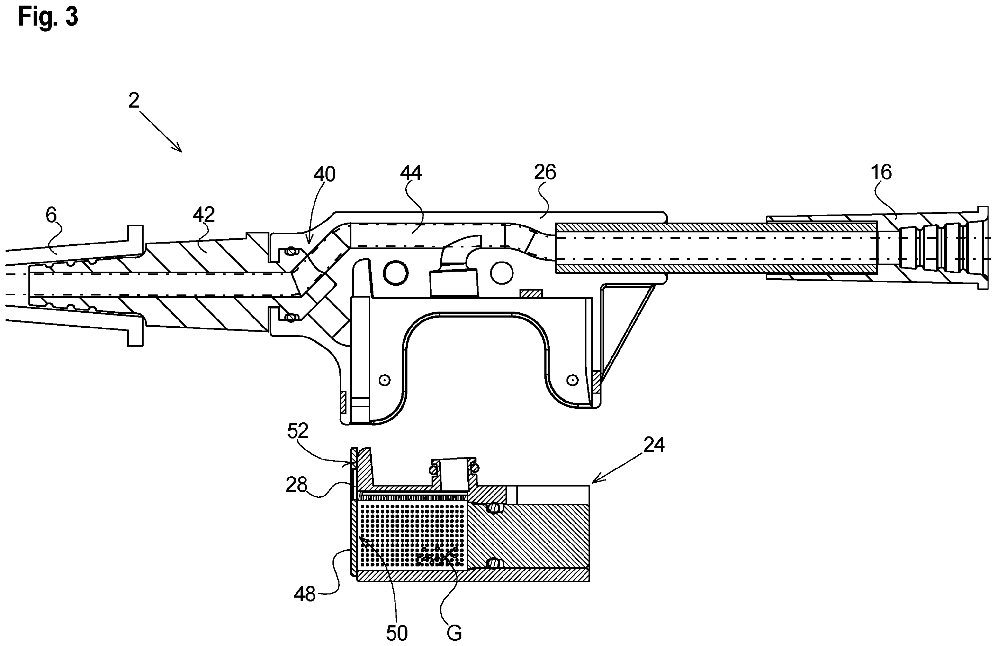

Durch Drehen des Stellgliedes 42 kann das Zweiwegeventil 40 in die Bypassstellung gemäß Figur 3 verbracht werden. In dieser ist der shaverseitige Strömungsanschluss 6 nun über einen Bypasspfad 44 mit dem unterdruckseitigen Strömungsanschluss 16 verbunden. Der Bypasspfad 44 ist hierbei getrennt vom Sammelbehälter 24 in das Anschlussgehäuse 26 integriert. Hierdurch kann die Sammelvorrichtung 2 zusammen mit der Gewebeentnahmevorrichtung 4 und der Unterdruckvorrichtung 20 in einem reinen Absaugbetrieb verwendet werden, der unabhängig von dem Sammelbehälter 24 erfolgt. Der Sammelbehälter 24 kann dadurch auch während des Betriebs der Gewebeentnahmevorrichtung 4, wie dargestellt, vom Anschlussgehäuse 26 abgenommen werden, um beispielsweise das hierin zuvor gesammelte Gewebematerial G zu entnehmen. Um hierbei die Saugleistung auch über den Bypasspfad 44 beibehalten zu können, kann an der Austrittsöffnung 30 auf Seite des Anschlussgehäuses 26 ein Verschlusselement vorgesehen sein (nicht dargestellt).

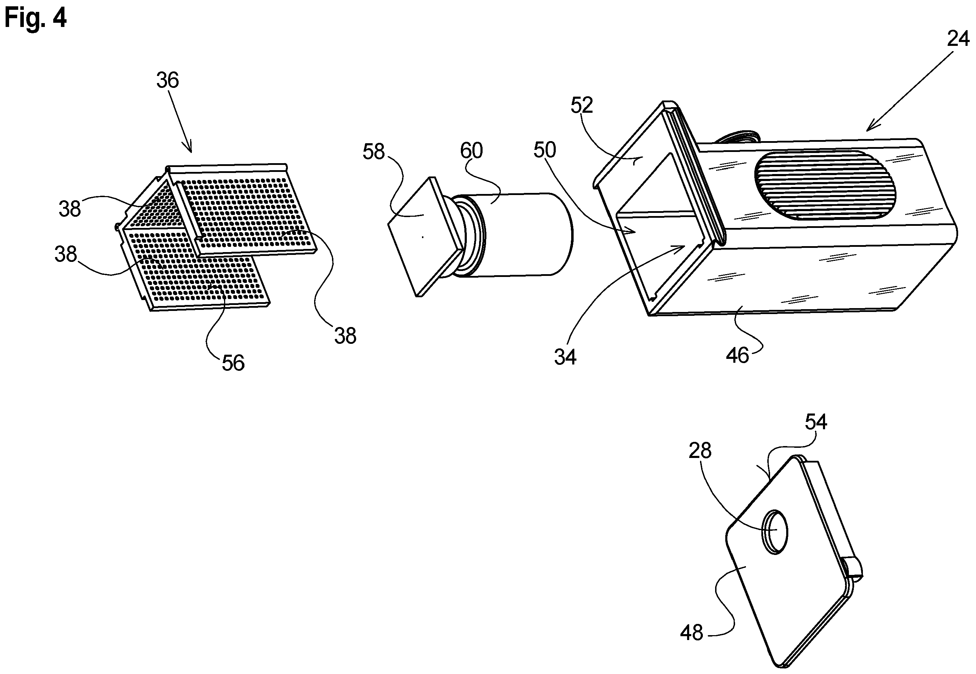

An dem Sammelbehälter 24 gemäß Figur 4 ist ein wenigstens teilweise transparenter Bereich 46 vorgesehen, über den die Sammelaufnahme 34 eingesehen werden kann. Der transparente Bereich 46 kann dabei, wie dargestellt, durch eine vollständige oder teilweise transparente Ausbildung des Sammelbehälters 24 selbst gebildet sein. Alternativ hierzu kann der transparente Bereich 46 auch durch ein Fenster gebildet sein, das in den ansonsten intransparent ausgebildeten Sammelbehälter 24 eingelassen ist (nicht dargestellt). Auf diese Weise kann überprüft werden, ob eine ausreichende Menge des abgetragenen Gewebematerials G gesammelt worden ist. Zur besseren Abschätzung der in der Sammelaufnahme 34 zurückgehaltenen Menge des Gewebematerials G kann hierbei am transparenten Bereich beziehungsweise in dem von diesem einsehbaren Bereich eine Messskala vorgesehen sein (nicht dargestellt).

Wie aus Figur 4 ferner zu entnehmen ist, ist die Zutrittsöffnung 28 der Sammelaufnahme 34 beispielsweise in ein Schließelement 48 integriert, das an einer Behälteröffnung 50 des Sammelbehälters 24 zwischen einer ersten und zweiten Offenstellung sowie einer Schließstellung verstellbar ist. In der Schließstellung befindet sich die Zutrittsöffnung 28 dabei auf Höhe einer geschlossenen Außenfläche 52 des Sammelbehälters 24, so dass die Sammelaufnahme 34 vom übrigen Schließelement 48 verschlossen wird, wie in Figur 3 dargestellt.

In der ersten Offenstellung befindet sich die Zutrittsöffnung 28 dagegen auf Höhe der Sammelaufnahme 34, so dass über diese der Verbindungspfad 14 ausgebildet werden kann, wie in Figur 2 dargestellt.

Das Schließelement 48 ist dabei derart ausgebildet, dass es beim Anbringen des Sammelbehälters 24 am Anschlussgehäuse 26 selbsttätig in die erste Offenstellung verbracht wird. Hierzu weist das Schließelement 48 ein Stellelement 54, das, wie dargestellt, beispielsweise durch einen Rand des Schließelementes 48 oder durch einen zusätzlich ausgeformten Kontaktnocken (nicht dargestellt) gebildet ist und das beim Anbringen des Sammelbehälters 24 am Anschlussgehäuse 26 in Anlage kommt und dadurch das Schließelement 48 von der Schließstellung in die Offenstellung verlagert.

Zusätzlich hierzu kann das Schließelement 48 beim Abnehmen vom Sammelbehälter selbsttätig in die Schließstellung verlagerbar sein. Hierzu kann zwischen dem Schließelement 48 und dem übrigen Sammelbehälter 24 beispielsweise eine Federeinrichtung vorgesehen sein (nicht dargestellt), die das Schließelement 48 in die Schließstellung vorspannt.

Wie aus Figur 4 ferner zu entnehmen ist, weist die Siebeinrichtung 36 beispielhaft drei ebene, Siebflächen 38 auf, entlang deren jeweiliger Innenoberfläche 56 ein Schaber 58 verlagerbar ist, der an einem Kolben 60 gehalten ist. Der Kolben 60 ist wiederum an einem von der Zutrittsöffnung 28 beziehungsweise vom Schließelement 48 abgewandten Ende der Sammelaufnahme 34 verschiebbar gehalten, wie aus den Figuren 2 und 3 zu entnehmen ist. Hierdurch kann der Kolben 60 entlang der Sammelaufnahme 34 in Richtung der Behälteröffnung 50 verschoben werden. Auf diese Weise kann in der zweiten Offenstellung des Schließelementes 48, in der dieses die Behälteröffnung 50 im Wesentlichen vollständig freigibt, das gesammelte Gewebematerial G vollständig aus der Sammelaufnahme 34 ausgegeben werden. Durch den am Kolben 60 mitgeführten Schaber 58 werden dabei auch an den Siebflächen 38 anhaftende Gewebepartikel gelöst und ebenfalls über die Behälteröffnung 50 ausgegeben, um eine im Wesentlichen vollständige Entleerung der Sammelaufnahme 34 sicherzustellen.

Es wird darauf hingewiesen, dass alle oben beschriebenen Elemente und Merkmale der verschiedenen Ausführungsformen des erfindungsgemäßen Gegenstandes untereinander austauschbar beziehungsweise kombinierbar sind, sofern ein Austausch oder eine Kombination derselben aus technischen Gründen nicht ausgeschlossen ist.

## Patentansprüche

1. Sammelvorrichtung (2) für eine Gewebeentnahmevorrichtung (4)
mit einem shaverseitigen Strömungsanschluss (6), der mit einer Gewebeabführung (12) der Gewebeentnahmevorrichtung (4) strömungsmäßig verbindbar ist,
mit einem unterdruckseitigen Strömungsanschluss (16), der mit einer Unterdruckvorrichtung (20) strömungsmäßig verbindbar ist,
einem Verbindungspfad (14), der zwischen dem shaverseitigen Strömungsanschluss (6) und dem unterdruckseitigen Strömungsanschluss (16) ausbildbar ist und über den ein Fluidstrom (F) vom shaverseitigen Strömungsanschluss (6) zum unterdruckseitigen Strömungsanschluss (16) ausbildbar ist, und
einem im Verbindungspfad (14) anbringbaren Sammelbehälter (24) zur Aufnahme von Gewebematerial (G) aus dem Fluidstrom (F),
wobei zwischen dem shaverseitigen Strömungsanschluss (6) und dem unterdruckseitigen Strömungsanschluss (16) ein vom Sammelbehälter (24) getrennter Bypasspfad (44) ausbildbar ist,
**dadurch gekennzeichnet, dass** der shaverseitige Strömungsanschluss (6) und der unterdruckseitige Strömungsanschluss (16) an einem Anschlussgehäuse (26) vorgesehen sind, an dem der Sammelbehälter (24) abnehmbar gehalten istund eine über eine Handhabe umschaltbare Umschalteinrichtung (40) vorgesehen ist, über die der shaverseitige Strömungsanschluss (6) wahlweise über den Verbindungspfad (14) oder den Bypasspfad (44) mit dem unterdruckseitigen Strömungsanschluss (16) verbindbar ist.

2. Sammelvorrichtung nach Anspruch 1, **dadurch gekennzeichnet, dass** die Umschalteinrichtung (40) durch ein Zweiwegeventil gebildet ist.

3. Sammelvorrichtung nach Anspruch 1 oder 2, **dadurch gekennzeichnet, dass** die Handhabe durch ein Drehglied (42) gebildet ist.

4. Sammelvorrichtung nach einem der Ansprüche 1 bis 3, **dadurch gekennzeichnet, dass** der Bypasspfad (44) innerhalb des Anschlussgehäuses (26) ausgebildet ist.

5. Sammelvorrichtung nach einem der Ansprüche 1 bis 4, **dadurch gekennzeichnet,**
**dass** der Sammelbehälter (24) zwischen einer Zutrittsöffnung (28) und einer Austrittsöffnung (30) einen Strömungsabschnitt (32) des Verbindungspfades (14) bildet, in dem eine durch eine Siebeinrichtung (36) begrenzte Sammelaufnahme (34) vorgesehen ist.

6. Sammelvorrichtung nach Anspruch 5, **dadurch gekennzeichnet, dass** die Siebeinrichtung (36) durch eine in Strömungsrichtung vor der Austrittsöffnung (30) angebrachte ebene Siebfläche (38) gebildet ist.

7. Sammelvorrichtung nach Anspruch 5 oder 6, **dadurch gekennzeichnet, dass** die Sammelaufnahme (34) über einen transparenten Bereich (46) des Sammelbehälters (24) einsehbar ist.

8. Sammelvorrichtung nach einem der Ansprüche 1 bis 7, **dadurch gekennzeichnet, dass** an dem abnehmbaren Sammelbehälter (24) ein Schließelement (48) gehalten ist, das zwischen einer ersten Offenstellung, in der die Zutrittsöffnung (28) der Sammelaufnahme (34) freigegeben ist, und einer Schließstellung verstellbar ist, in der die Zutrittsöffnung (28) verschlossen ist.

9. Sammelvorrichtung nach Anspruch 8, **dadurch gekennzeichnet, dass** das Schließelement (48) beim Anbringen des Sammelbehälters (24) am Anschlussgehäuse (26) selbsttätig in die erste Offenstellung verbringbar ist.

10. Sammelvorrichtung nach Anspruch 8 oder 9, **dadurch gekennzeichnet, dass** das Schließelement (48) beim Abnehmen vom Sammelbehälter (24) selbsttätig in die Schließstellung verbringbar ist.

11. Sammelvorrichtung nach einem der Ansprüche 8 bis 10, **dadurch gekennzeichnet, dass** das Schließelement (48) ein Stellelement (54) aufweist, über das das Schließelement (48) beim Anbringen des Sammelbehälters (24) durch Zusammenwirken mit dem Anschlussgehäuse (26) in die erste Offenstellung verstellbar ist.

12. Sammelvorrichtung nach einem der Ansprüche 8 bis 11, **dadurch gekennzeichnet, dass** das Schließelement (48) in die Schließstellung vorgespannt ist.

13. Sammelvorrichtung nach einem der Ansprüche 5 bis 12, **dadurch gekennzeichnet, dass** die Sammelaufnahme (34) mittels eines Kolbens (60) entleerbar ist.

14. Sammelvorrichtung nach Anspruch 13, **dadurch gekennzeichnet, dass** der Sammelbehälter (24) eine vom Schließelement (48) verschließbare Behälteröffnung (50) aufweist, die in einer zweiten Offenstellung des Schließelementes (48) freigegeben ist, und der Kolben (60) an einer vom Schließelement (48) abgewandten Seite der Sammelaufnahme (34) gehalten und entlang der Sammelaufnahme (34) in Richtung der Behälteröffnung (50) verlagerbar ist.

15. Sammelvorrichtung nach Anspruch 13 oder 14, **dadurch gekennzeichnet, dass** der Kolben (60) entlang einer Innenoberfläche (56) der Siebeinrichtung (36) verlagerbar ist.

## Claims

1. Collecting device (2) for a tissue biopsy device (4)
with a shaver-side flow connection (6), which can be fluidly connected to a tissue discharge (12) of the tissue biopsy device (4),
with a vacuum-side flow connection (16), which can be fluidly connected to a vacuum device (20),
a connection path (14), which can be formed between the shaver-side flow connection (6) and the vacuum-side flow connection (16) and through which a fluid flow (F) can be formed from the shaver-side flow connection (6) to the vacuum-side flow connection (16), and
a collection container (24), which can be mounted in the connection path (14) to receive tissue material (G) from the fluid flow (F),
wherein a bypass path (44) separated from the collection container (24) can be formed between the shaver-side flow connection (6) and the vacuum-side flow connection (16),
**characterized in that** the shaver-side flow connection (6) and the vacuum-side flow connection (16) are provided on a connection housing (26), on which the collection container (24) is detachably held, and a switching apparatus (40), which can be switched using a handle, is provided, by means of which the shaver-side flow connection (6) can be optionally connected via the connection path (14) or the bypass path (44) to the vacuum-side flow connection (16).

2. Collecting device according to claim 1, **characterized in that** the switching apparatus (40) is formed by a two-way valve.

3. Collecting device according to claim 1 or 2, **characterized in that** the handle is formed by a rotary member (42).

4. Collecting device according to any one of claims 1 to 3, **characterized in that** the bypass path (44) is formed within the connection housing (26).

5. Collecting device according to any one of claims 1 to 4, **characterized in that** the collection container (24) forms a flow section (32) of the connection path (14) between an inlet opening (28) and an outlet opening (30), in which a collection receptacle (34) bounded by a screening apparatus (36) is provided.

6. Collecting device according to claim 5, **characterized in that** the screening apparatus (36) is formed by a planar screen surface (38) mounted upstream of the outlet opening (30).

7. Collecting device according to claim 5 or 6, **characterized in that** the collection receptacle (34) is visible via a transparent region (46) of the collection container (24).

8. Collecting device according to any one of claims 1 to 7, **characterized in that** a closing element (48) is held on the detachable collection container (24), which closing element (48) is adjustable between a first open position, in which the inlet opening (28) of the collection receptacle (34) is unlocked, and a closed position, in which the inlet opening (28) is locked.

9. Collecting device according to claim 8, **characterized in that** the closing element (48) can be automatically moved into the first open position on mounting the collection container (24) on the connection housing (26).

10. Collecting device according to claim 8 or 9, **characterized in that** the closing element (48) can be automatically moved into the closed position on removal from the collection container (24).

11. Collecting device according to any one of claims 8 to 10, **characterized in that** the closing element (48) has an actuating element (54), by means of which the closing element (48) can be moved into the first open position on mounting the collection container (24) through interaction with the connection housing (26).

12. Collecting device according to any one of claims 8 to 11, **characterized in that** the closing element (48) is prestressed in the closed position.

13. Collecting device according to any one of claims 5 to 12, **characterized in that** the collection receptacle (34) can be emptied by means of a plunger (60).

14. Collecting device according to claim 13, **characterized in that** the collection container (24) has a container opening (50) that can be closed by the closing element (48), which is unlocked in a second open position of the closing element (48), and the plunger (60) is held on a side of the collection receptacle (34) facing away from the closing element (48) and can be displaced along the collection receptacle (34) in the direction of the container opening (50).

15. Collecting device according to claim 13 or 14, **characterized in that** the plunger (60) can be displaced along an inner surface (56) of the screening apparatus (36).

## Revendications

1. Dispositif collecteur (2) pour un dispositif de prélèvement de tissu (4)
avec un raccord d'écoulement (6) côté shaver qui peut être relié en communication fluidique à une évacuation de tissu (12) du dispositif de prélèvement de tissu (4),
avec un raccord d'écoulement (16) côté dépression qui peut être relié en communication fluidique à un dispositif de dépression (20),
un chemin de liaison (14) qui peut être formé entre le raccord d'écoulement (6) côté shaver et le raccord d'écoulement (16) côté dépression et par l'intermédiaire duquel un écoulement de fluide (F) peut être formé du raccord d'écoulement (6) côté shaver au raccord d'écoulement (16) côté dépression, et
un récipient collecteur (24) pouvant être monté dans le chemin de liaison (14) pour recevoir le matériau de tissu (G) provenant de l'écoulement de fluide (F),
dans lequel un chemin de dérivation (44) séparé du récipient collecteur (24) peut être formé entre le raccord d'écoulement (6) côté shaver et le raccord d'écoulement (16) côté dépression,
**caractérisé en ce que** le raccord d'écoulement (6) côté shaver et le raccord d'écoulement (16) côté dépression sont prévus sur un boîtier de raccordement (26) sur lequel le récipient collecteur (24) est maintenu de manière amovible et un appareil de commutation (40) commutable par une poignée est prévu, appareil par l'intermédiaire duquel le raccord d'écoulement (6) côté shaver peut être relié au raccord d'écoulement (16) côté dépression au choix par l'intermédiaire du chemin de liaison (14) ou du chemin de dérivation (44).

2. Dispositif collecteur selon la revendication 1, **caractérisé en ce que** l'appareil de commutation (40) est formé par une vanne à deux voies.

3. Dispositif collecteur selon la revendication 1 ou 2, **caractérisé en ce que** la poignée est formée par un membre rotatif (42).

4. Dispositif collecteur selon l'une quelconque des revendications 1 à 3, **caractérisé en ce que** le chemin de dérivation (44) est formé à l'intérieur du boîtier de raccordement (26).

5. Dispositif collecteur selon l'une quelconque des revendications 1 à 4, **caractérisé en ce que** le récipient collecteur (24) forme, entre une ouverture d'accès (28) et une ouverture de sortie (30), une section d'écoulement (32) du chemin de liaison(14), dans lequel un réceptacle collecteur (34) délimité par un appareil de tamisage (36) est prévu.

6. Dispositif collecteur selon la revendication 5, **caractérisé en ce que** l'appareil de tamisage (36) est formé par une surface de tamisage plane (38) placée en amont de l'ouverture de sortie (30) dans le sens d'écoulement.

7. Dispositif collecteur selon la revendication 5 ou 6, **caractérisé en ce que** le réceptacle collecteur (34) est visible par l'intermédiaire d'une zone transparente (46) du récipient collecteur (24).

8. Dispositif collecteur selon l'une quelconque des revendications 1 à 7, **caractérisé en ce qu'**un élément de fermeture (48) est maintenu sur le récipient collecteur amovible (24), élément qui est réglable entre une première position d'ouverture dans laquelle l'ouverture d'accès (28) du réceptacle collecteur (34) est libérée et une position de fermeture dans laquelle l'ouverture d'accès (28) est fermée.

9. Dispositif collecteur selon la revendication 8, **caractérisé en ce que** l'élément de fermeture (48) peut être placé automatiquement dans la première position d'ouverture lorsque le récipient collecteur (24) est placé sur le boîtier de raccordement (26).

10. Dispositif collecteur selon la revendication 8 ou 9, **caractérisé en ce que** l'élément de fermeture (48) peut être placé automatiquement dans la position de fermeture lorsqu'il est retiré du récipient collecteur (24).

11. Dispositif collecteur selon l'une quelconque des revendications 8 à 10, **caractérisé en ce que** l'élément de fermeture (48) présente un élément de réglage (54) par l'intermédiaire duquel l'élément de fermeture (48) peut être déplacé dans la première position d'ouverture lorsque le récipient collecteur (24) est placé par interaction avec le boîtier de raccordement (26).

12. Dispositif collecteur selon l'une quelconque des revendications 8 à 11, **caractérisé en ce que** l'élément de fermeture (48) est précontraint dans la position de fermeture.

13. Dispositif collecteur selon l'une quelconque des revendications 5 à 12, **caractérisé en ce que** le réceptacle collecteur (34) peut être vidé au moyen d'un piston (60).

14. Dispositif collecteur selon la revendication 13, **caractérisé en ce que** le récipient collecteur (24) présente une ouverture de récipient (50) pouvant être fermée par l'élément de fermeture (48), ouverture qui est libérée dans une seconde position d'ouverture de l'élément de fermeture (48), et le piston (60) est maintenu sur un côté du réceptacle collecteur (34) opposé à l'élément de fermeture (48) et peut être déplacé le long du réceptacle collecteur (34) en direction de l'ouverture de récipient (50).

15. Dispositif collecteur selon la revendication 13 ou 14, **caractérisé en ce que** le piston (60) peut être déplacé le long d'une surface interne (56) de l'appareil de tamisage (36).
